(19) Europäisches Patentamt
European Patent Office
Office européen des brevets

(11) Veröffentlichungsnummer: **0 076 917**
**B1**

(12) # EUROPÄISCHE PATENTSCHRIFT

(45) Veröffentlichungstag der Patentschrift: **17.04.85**

(51) Int. Cl.⁴: **C 07 C 103/46**, C 07 C 103/48, C 07 C 149/43, B 01 J 23/36 // C07C117/08

(21) Anmeldenummer **82107926.6**

(22) Anmeldetag: **28.08.82**

(54) **Verfahren zur Herstellung von N-Acetyl-2,3-dehydro-aminocarbonsäureestern.**

(30) Priorität: **09.10.81 DE 3140227**

(43) Veröffentlichungstag der Anmeldung: **20.04.83 Patentblatt 83/16**

(45) Bekanntmachung des Hinweises auf die Patenterteilung: **17.04.85 Patentblatt 85/16**

(84) Benannte Vertragsstaaten: **AT BE CH DE FR GB IT LI NL**

(56) Entgegenhaltungen:
**ANGEWANDTE CHEMIE, Band 94, Nr. 3, März 1982, Weinheim**
**JOURNAL OF ORGANIC CHEMISTRY, Band 45, Nr. 24, Dezember 1980**
**Chemical Abstracts Band 69, Nr. 25, 16. Dezember 1968, Columbus, Ohio, USA**

(73) Patentinhaber: **Degussa Aktiengesellschaft, Weissfrauenstrasse 9, D-6000 Frankfurt am Main 1 (DE)**

(72) Erfinder: **Effenberger, Franz, Dr. Prof., Schatzweg 5, *D-7000 Stuttgart 70* (DE)**
Erfinder: **Beisswenger, Thomas, Dipl.-Chem., Hessenlauweg 19, D-7000 Stuttgart 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 076 917 B1

**Beschreibung**

Gegenstand der Erfindung ist ein Verfahren zur Herstellung von N-Acetyl-2,3-dehydro-aminocarbonsäureestern der allgemeinen Formel

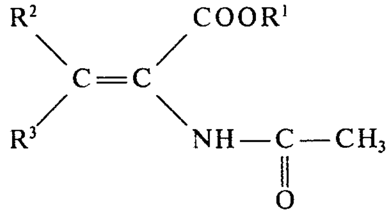

(I)

in der $R^1$ einen Methyl- oder Ethylrest, $R^2$ Wasserstoff oder einen Methylrest und $R^3$ Wasserstoff, einen Alkylrest mit 1 bis 6 Kohlenstoffatomen, einen Arylrest, einen unsubstituierten oder substituierten Alkylmercaptorest oder einen Arylmercaptorest bedeuten.

Verbindungen der allgemeinen Formel (I) sind bekannt. Sie dienen hauptsächlich als Zwischenprodukte für die Herstellung von optisch aktiven 2-N-Acetyl-aminocarbonsäuren durch asymmetrische katalytische Hydrierung der prochiralen C=C-Doppelbindung.

Es ist bereits bekannt, N-Acetyl-2,3-dehydroaminocarbonsäureester in einem zweistufigen Verfahren durch Umsetzung von 2-Azido-carbonsäureestern mit n-Butyllithium/Ethanol und anschließende Acylierung mit Acetylchlorid herzustellen (J. Org. Chem., Vol. 45, 1980, Seiten 4952 bis 4954). Auf diese Weise läßt sich z. B. der N-Acetyl-2,3-dehydro-alaninethylester in einer Ausbeute von 58% der Theorie herstellen.

Das erfindungsgemäße Verfahren ist nun daduch gekennzeichnet, daß man einen 2-Azido-carbonsäureester der allgemeinen Formel

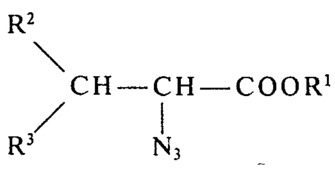

(II)

in der $R^1$, $R^2$ und $R^3$ die bereits angegebene Bedeutung aufweisen, in Gegenwart von Rhenium-VII-sulfid und/oder -oxid und bei einer Temperatur zwischen 50 und 150 °C mit einem Gemisch aus einem Volumenteil Essigsäureanhydrid und 1,5 bis 5 Volumteilen Essigsäure umsetzt.

Auf diese Weise lassen sich die gewünschten N-Acetyl-2,3-dehydro-aminocarbonsäureester der allgemeinen Formel (I) in einem einstufigen Verfahren leicht und in hoher Ausbeute herstellen

Beispiele für nach dem erfindungsgemäßen Verfahren umzusetzende 2-Azido-carbonsäureester der allgemeinen Formel (II) sind unter anderem die Methyl- und Ethylester von

2-Azido-propionsäure, 2-Azido-buttersäure, 2-Azido-3-methyl-buttersäure,
2-Azido-3-phenyl-propionsäure, 2-Azido-valeriensäure, 2-Azido-4-methyl-pentansäure,
2-Azido-hexansäure, 2-Azido--heptansäure, 2-Azido-3-methylmercapto, propionsäure,
2-Azido-3-methoxycarbonylmethylmercapto-propionsäure oder
2-Azido-3-phenylmercapto-propionsäure.

Das als Katalysator dienende Rhenium-VII-sulfid und/oder -oxid wird zweckmäßigerweise in einer Menge zwischen 0,01 und 10 Molprozent, bezogen auf den eingesetzten 2-Azido-carbonsäureester der allgemeinen Formel (II), angewandt. Die besonders bevorzugten Anwendungsmengen liegen im Falle des Rhenium-VII-sulfids zwischen 0,5 und 2 Molprozent und im Falle des Rhenium-VII-oxids zwischen 0,05 und 1 Molprozent.

Das erfindungsgemäße Verfahren wird vorzugsweise bei einer Temperatur zwischen 60 und 90 °C durchgeführt. Um Ausbeuteverluste durch Polymerisation der entstehenden N-Acetyl-2,3-dehydroaminocarbonsäureester der allgemeinen Formel (I) zu vermeiden, ist es zweckmäßig, die Umsetzung in Gegenwart eines bekannten Inhibitors für radikalische Polymerisationen, wie Hydrochinon oder Hydrochinon-monomethylether, vorzunehmen. Als Zusatzmenge für den Inhibitor kommen 0,001 bis 10 Gewichtsprozent, insbesondere 0,1 bis 2,5 Gewichtsprozent, bezogen auf den eingesetzten 2-Azidocarbonsäureester der allgemeinen Formel (II), in Frage.

Das als Acetylierungsmittel dienende Gemisch aus einem Volumteil Essigsäureanhydrid und 1,5 bis 5 Volumteilen Essigsäure wird zweckmäßig in einer Menge von 260 bis 5000 ml, vorzugsweise von 500 bis 3000 ml, pro Mol eingesetzten 2-Azido-carbonsäureesters der allgemeinen Formel (II) angewandt.

Das erfindungsgemäße Verfahren kann beispielsweise so durchgeführt werden, daß man das Gemisch aus Essigsäureanhydrid und Essigsäure mit dem Rhenium-VII-sulfid und/oder -oxid und gegebenenfalls dem Polymerisationsinhibitor vorlegt und unter kräftigem Rühren den umzusetzenden 2-Azido-carbonsäureester der allgemeinen Formel (II) langsam, z. B. im Verlauf von 2 Stunden, zudo-

siert. Es ist empfehlenswert, das Reaktionsgemisch nach beendeter Stickstoffentwicklung noch längere Zeit, beispielsweise 20 Stunden, auf der Reaktionstemperatur zu halten.

Eine beträchtliche Verkürzung der erforderlichen Reaktionszeit läßt sich in vielen Fällen erzielen, wenn man die Umsetzung in gleichzeitiger Gegenwart von trockenem Chlorwasserstoff vornimmt. In diesem Falle ist es vorteilhaft, den umzusetzenden 2-Azido-carbonsäureester der allgemeinen Formel (II) in dem Gemisch aus Essigsäureanhydrid und Essigsäure zu lösen, das Rhenium-VII-sulfid und/oder -oxid und gegebenenfalls den Polymerisationsinhibitor zuzusetzen und dann trockenen Chlorwasserstoff bis zur Sättigung einzuleiten. Anschließend wird dann auf die Reaktionstemperatur erhitzt. Im allgemeinen ist bei dieser Arbeitsweise eine Reaktionszeit von höchstens 3 Stunden ausreichend.

Nach beendeter Umsetzung werden die Essigsäure und das überschüssige Essigsäureanhydrid, zweckmäßigerweise unter vermindertem Druck, beispielsweise in einem Rotationsverdampfer, entfernt. Der Rückstand wird in einem leichtflüchtigen Lösungsmittel, beispielsweise Diethylether, aufgenommen, die Lösung filtriert und das Filtrat unter vermindertem Druck eingedampft. Zur weiteren Reinigung wird der Rückstand dann mit einem Gemisch aus niedrigsiedendem Petrolether und Essigsäureethylether (Volumenverhältnis etwa 2 : 1) als Laufmittel über eine Kieselgelsäule chromatographiert. Nach Eindampfen des Eluats, zweckmäßigerweise wieder unter vermindertem Druck, hinterbleibt der praktisch analysenreine N-Acetyl-2,3-dehydro-aminocarbonsäureester der allgemeinen Formel (I).

Aus den N-Acetyl-2,3-dehydro-aminocarbonsäureestern können dann durch asymmetrische katalytische Hydrierung in bekannter Weise wahlweise die entsprechenden L- oder D-N-Acetyl-aminocarbonsäureester hergestellt und diese zu den entsprechenden L- oder D-2-Amino-carbonsäuren verseift werden.

Die Erfindung wird durch die nachfolgenden Beispiele näher erläutert:

## Beispiel 1

0,925 g (1,55 mMol) Rhenium-VII-sulfid und 0,5 g Hydrochinon werden in einem Gemisch aus 120 ml Essigsäureanhydrid und 280 ml Essigsäure gelöst. Dann werden bei 80°C innerhalb von 2 Stunden unter kräftigem Rühren 20,0 g (0,155 Mol) 2-Azido-propionsäuremethylester zugetropft.

Die Umsetzung verläuft unter gleichmäßiger Stickstoffentwicklung. Nach dem Ende der Gasentwicklung läßt man noch 20 Stunden bei 80°C nachreagieren und entfernt dann die Essigsäure und das überschüssige Essigsäureanhydrid unter vermindertem Druck. Der Rückstand wird in 100 ml Diethylether aufgenommen, die Lösung filtriert, das Filtrat eingedampft und der Rückstand über eine 20 cm hohe Kieselgelsäule mit einem Gemisch aus niedrigsiedendem Petrolether und Essigsäureethylester im Volumenverhältnis 2 : 1 als Laufmittel chromatographiert.

Nach dem Eindampfen des Eluats erhält man 15,7 g (71% der Theorie) analysenreinen N-Acetyl-2,3-dehydro-alaninmethylester

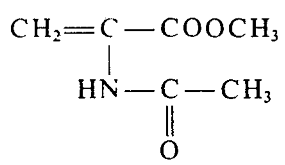

mit einem Schmelzpunkt von 52°C (Literatur: 52—54°C).

$C_6H_9NO_3$(143, 14)

| | | | |
|---|---|---|---|
| Berechnet: | C 50,35% | H 6,34% | N 9,79% |
| Gefunden: | C 50,30% | H 6,27% | N 9,51% |

## Beispiel 2

1,0 g (5,84 mMol) 2-Azido-hexansäuremethylester werden in einem Gemisch aus 2 ml Essigsäureanhydrid und 3 ml Essigsäure gelöst und mit 5 mg Hydrochinon und 35 mg Rhenium-VII-sulfid versetzt. Dann wird die Lösung mit trockenem Chlorwasserstoff gesättigt.

Das Reaktionsgemisch wird unter kräftigem Rühren 2 Stunden lang auf 80°C gehalten und anschließend analog zu Beispiel 1 aufgearbeitet. Man erhält 0,98 g (91% der Theorie) analysenreinen N-Acetyl-2,3-dehydro-norleucinmethylester mit einem Schmelzpunkt von 49—51°C.

$C_9H_{15}NO_3$(185, 22)

| | | | |
|---|---|---|---|
| Berechnet: | C 58,36% | H 8,16% | N 7,56% |
| Gefunden: | C 58,18% | H 8,25% | N 7,48% |

$^1$H-NMR ($CDCl_3$): $\delta$ = 7,5 (s, 1H) NH;
7,0 (t, 1H) CH;
3,83 (s, 3H) $COOCH_3$;
2,52 (q, 2H) $CH_2-CH=$;
2,08 (s, 3H) $N-COCH_3$;
1,49 (m, 2H) $CH_3-CH_2-CH_2$;
0,94 ppm (t, 3H) $CH_3-CH_2$.

## Beispiel 3

1,0 g (4,56 mMol) 2-Azido-3-phenyl-propionsäureethylester werden in einem Gemisch aus 1 ml Essigsäureanhydrid und 4 ml Essigsäure gelöst und mit 5 mg Hydrochinon und 27 mg Rhenium-VII-sulfid versetzt. Dann wird die Lösung mit trockenem Chlorwasserstoff gesättigt.

Das Reaktionsgemisch wird unter kräftigem Rühren 2,5 Stunden lang auf 80°C gehalten und anschließend analog zu Beispiel 1 aufgearbeitet. Man erhält 0,96 g (90% der Theorie) analysenreinen N-Acetyl-2,3-dehydro-phenylalaninethylester

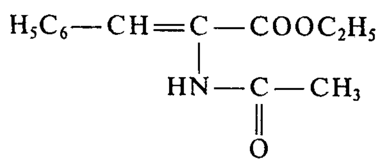

mit einem Schmelzpunkt von 96—97,5°C (Literatur: 96—98°C).

$C_{13}H_{15}NO_3$(233, 267)

| | | | |
|---|---|---|---|
| Berechnet: | C 66,94% | H 6,48% | N 6,01% |
| Gefunden: | C 66,77% | H 6,62% | N 5,72% |

$^1$H-NMR ($CDCl_3$): $\delta$ = 9,0 (s, 1H) NH;
7,30—7,75 (m, 5H) arom.-CH=;
4,26 (q, 2H) $COOCH_2$;
1,32 (t, 3H) $COOCH_2-CH_3$;
2,06 ppm (s, 3H) $CO-CH_3$.

## Beispiel 4

1,0 g (6,36 mMol) 2-Azido-3-methyl-buttersäuremethylester werden in einem Gemisch aus 2 ml Essigsäureanhydrid und 4 ml Essigsäure gelöst und mit 5 mg Hydrochinon und 38 mg Rhenium-VII-sulfid versetzt. Dann wird die Lösung mit trockenem Chlorwasserstoff gesättigt.

Das Reaktionsgemisch wird unter kräftigem Rühren 2 Stunden lang auf 80°C gehalten und anschließend analog zu Beispiel 1 aufgearbeitet. Man erhält 0,98 g (91% der Theorie) analysenreinen N-Acetyl-2,3-dehydro-valinmethylester

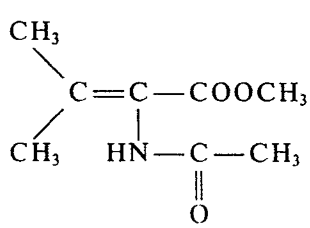

mit einem Schmelzpunkt von 93—94°C (Literatur: 88—89°C).

$C_8H_{13}NO_3$ (171,196)

| | | | |
|---|---|---|---|
| Berechnet: | C 56,13% | H 7,65% | N 8,18% |
| Gefunden: | C 56,10% | H 7,56% | N 8,28% |

$^1H$—NMR ($CDCl_3$): δ = 7,55 (s, 1 H) NH;
3,72 (s, 3 H) $COOCH_3$;
2,15 (d, 3 H) 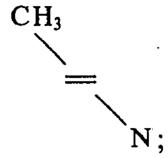
2,06 (s, 3 H) $CO—CH_3$;
1,83 ppm (s, 3 H) 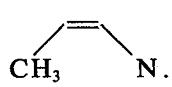

Beispiel 5

1,0 g (6,36 mMol) 2-Azido-3-methyl-buttersäuremethylester werden in einem Gemisch aus 2 ml Essigsäureanhydrid und 3 ml Essigsäure gelöst und mit 10 mg Hydrochinon und 15,4 mg Rhenium-VIIoxid versetzt. Dann wird die Lösung mit trockenem Chlorwasserstoff gesättigt.

Das Reaktionsgemisch wird unter kräftigem Rühren 10 Minuten lang auf 80° C gehalten und anschließend analog zu Beispiel 1 aufgearbeitet. Man erhält 0,97 g (90% der Theorie) analysenreinen N-Acetyl-2,3-dehydro-valinmethylester.

Beispiel 6

1,0 g (6,36 mMol) 2-Azido-3-methyl-buttersäuremethylester werden in einem Gemisch aus 2 ml Essigsäureanhydrid und 3 ml Essigsäure gelöst und mit 10 mg Hydrochinon und 1,5 mg Rhenium-VIIoxid versetzt. Dann wird die Lösung mit tockenem Chlorwasserstoff gesättigt.

Das Reaktionsgemisch wird unter kräftigem Rühren 2 Stunden lang auf 80° C gehalten und anschließend analog zu Beispiel 1 aufgearbeitet. Man erhält 0,97 g (90% der Theorie) analysenreinen N-Acetyl-2,3-dehydro-valinmethylester.

Beispiel 7

1,0 g (4,21 mMol) 2-Azido-3-phenylmercapto-propionsäure-methylester werden in einem Gemisch aus 1,5 ml Essigsäureanhydrid und 3,5 ml Essigsäure gelöst und mit 5 mg Hydrochinon und 25 mg Rhenium-VII-sulfid versetzt. Dann wird die Lösung mit trockenem Chlorwasserstoff gesättigt.

Das Reaktionsgemisch wird unter kräftigem Rühren 3 Stunden lang auf 85° C gehalten und anschließend analog zu Beispiel 1 aufgearbeitet. Man erhält 0,82 g (77% der Theorie) N-Acetyl-3-phenylmercapto-2,3-dehydro-alaninmethylester

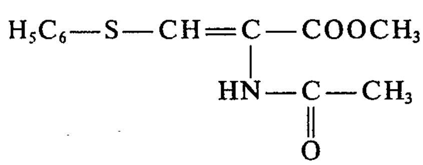

mit einem Schmelzpunkt von 103—105° C.

$C_{12}H_{13}NO_3S$ (251, 304)

| | | | | |
|---|---|---|---|---|
| Berechnet: | C 57,35% | H 5,21% | N 5,57% | S 12,76% |
| Gefunden: | C 57,19% | H 5,22% | N 5,66% | S 12,90% |

$^1H$-NMR ($CDCl_3$): $\delta$ = 8,06 (s, 1H) NH;
7,2—7,6 (m, 5H) arom.-CH =;
7,66 (s, 1H) S—CH =;
3,78 (s, 3H) $COOCH_3$;
2,18 ppm (s, 3H) $CO—CH_3$.

**Patentansprüche**

1. Verfahren zur Herstellung von N-Acetyl-2,3-dehydro-aminocarbonsäureestern der allgemeinen Formel

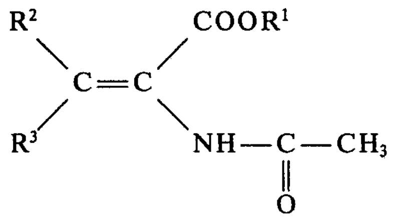

(I)

in der $R^1$ einen Methyl- oder Ethylrest, $R^2$ Wasserstoff oder einen Methylrest und $R^3$ Wasserstoff, einen Alkylrest mit 1 bis 6 Kohlenstoffatomen, einen Arylrest, einen unsubstituierten oder substituierten Alkylmercaptorest oder einen Arylmercaptorest bedeuten, dadurch gekennzeichnet, daß man einen 2-Azido-carbonsäureester der allgemeinen Formel

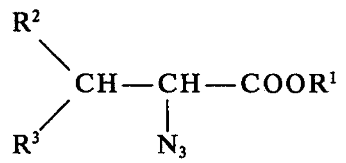

(II)

in der $R^1$, $R^2$ und $R^3$ die bereits angegebene Bedeutung aufweisen, in Gegenwart von Rhenium-VII-sulfid und/oder -oxid und bei einer Temperatur zwischen 50 und 150°C mit einem Gemisch aus einem Volumteil Essigsäureanhydrid und 1,5 bis 5 Volumteilen Essigsäure umsetzt.

2. Verfahren nach Anspruch 1, dadurch gekennzeichnet, daß man die Umsetzung in gleichzeitiger Gegenwart von trockenem Chlorwasserstoff vornimmt.

3. Verfahren nach Anspruch 1 oder 2, dadurch gekennzeichnet, daß man die Umsetzung in gleichzeitiger Gegenwart eines bekannten Inhibitors für radikalische Polymerisationen vornimmt.

4. Verfahren nach einem der Ansprüche 1 bis 3, dadurch gekennzeichnet, daß man das Rhenium-VII-sulfid und/oder -oxid in einer Menge zwischen 0,01 und 10 Molprozent, bezogen auf den eingesetzten 2-Azido-carbonsäureester der allgemeinen Formel (II), anwendet.

**Claims**

1. Process for the production of N-acetyl-2,3-dehydroaminocarboxylic acid esters corresponding to the general formula

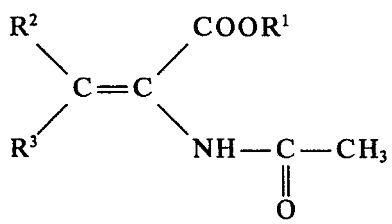

(I)

wherein,
$R^1$ represents a methyl or ethyl radical,
$R^2$ represents hydrogen or a methyl radical, and
$R^3$ represents hydrogen, an alkyl radical having from 1 to 6 carbon atoms, an aryl radical, an unsubstituted or substituted alkylmercapto radical or an arylmercapto radical,
characterised in that, a 2-azido-carboxylic acid ester corresponding to the general formula

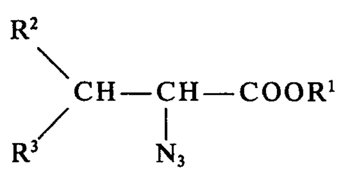

(II)

wherein,
$R^1$, $R^2$ and $R^3$ are as defined above,
is reacted in the presence of rhenium-VII-sulphide and/or -oxide and at a temperature of from 50°C to

150° C with a mixture consisting of one part by volume acetic acid anhydride and from 1.5 to 5 parts by volume acetic acid.

2. Process according to claim 1, characterised in that the reaction is carried out in addition in the presence of dry hydrochloric acid.

3. Process according to claim 1 or claim 2, characterised in that the reaction is carried out in addition in the presence of a known inhibitor for radical polymerisation.

4. Process according to one of claims 1 to 3, characterised in that the rhenium-VII-sulphide and/or oxide is used in a quantity of from 0.01 to 10 mol%, based on the 2-azido-carboxylic acid ester according to the general formula (II) used.

**Revendications**

1. Procédé pour la fabrication d'esters d'acides N-acétyl-2,3-déhydro-amino-carboxylique répondant à la formule générale:

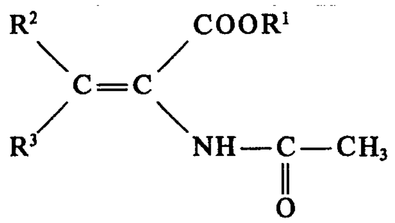

(I)

où $R^1$ représente un reste méthyle ou éthyle, $R^2$ un hydrogène, ou un reste méthyle, et $R^3$ un hydrogène, un reste alcoyle à 1 à 6 atomes de carbone, un reste aryle, un reste alcoylmercapto, substitué ou non, ou un reste arylmercapto, caractérisé en ce qu'on fait réagir un ester d'acide 2-azido-carboxylique de formule générale:

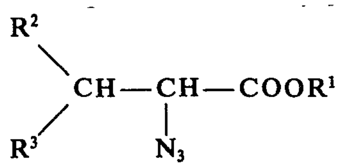

(II)

où $R^1$, $R^2$, et $R^3$ ont la même signification qui est indiquée plus haut, en présence de sulfure et/ou d'oxyde de rhénium-VII, et à une température de l'ordre de 50 à 150° C, avec un mélange d'une partie en volume d'anhydride acétique, et 1,5 à 5 parties en volume d'acide acétique.

2. Procédé suivant la revendication 1, caractérisé en ce que l'on assure la réaction en présence simultanée d'acide chlorhydrique sec.

3. Procédé suivant l'une des revendications 1 ou 2, caractérisé en ce que l'on assure la réaction en présence simultanée d'un inhibiteur des polymérisations radicalaires connu.

4. Procédé suivant l'une des revendications 1 à 3, caractérisé en ce que l'on utilise le sulfure et/ou l'oxyde de rhénium-VII dans une proportion qui se situe entre 0,01 et 10 mol% calculé sur l'ester de l'acide 2-azido-carboxylique de formule générale (II) mis en œuvre.